# EUROPEAN PATENT APPLICATION

(11) **EP 4 425 959 A1**
(43) Date of publication of application: **04.09.2024**
(21) Application number: 22886977.2
(22) Date of filing: 25.10.2022
(51) Int. Cl.: H04R 1/34, H04R 17/00

(54) **ULTRASONIC WAVE GENERATING DEVICE AND ULTRASONIC WAVE GENERATING SYSTEM**

(30) Priority: 27.10.2021 JP 2021175394
(71) Applicant: Niterra Co., Ltd., Nagoya-shi, Aichi 461-0005 (JP)
(72) Inventor: SUZUKI Satoshi, Nagoya-shi, Aichi 461-0005 (JP); YOKOYAMA Kota, Nagoya-shi, Aichi 461-0005 (JP); SUZUKI Ryo, Nagoya-shi, Aichi 461-0005 (JP); ITOH Shinsuke, Nagoya-shi, Aichi 461-0005 (JP); KASASHIMA Takashi, Nagoya-shi, Aichi 461-0005 (JP)
(74) Representative: Grünecker Patent- und Rechtsanwälte PartG mbB
(86) International application number: PCT/JP2022/039607
(87) International publication number: WO 2023/074650

(57) **Abstract**

Provided are an ultrasonic wave generation device and an ultrasonic wave generation system that can inhibit the intensity of an ultrasonic wave from decreasing owing to an adhesive. A waveguide (13) is a member separate from an ultrasonic wave converging part (12). The ultrasonic wave converging part (12) is adhered to an ultrasonic wave generation source (11) by using an adhesive (20). When a line obtained by projecting an outer circumferential edge (11B) of the ultrasonic wave generation source (11) onto a first reflection surface (16) along an advancing direction of an ultrasonic wave generated from the ultrasonic wave generation source (11) is defined as a first imaginary line (L1), and straight lines passing through points on the first imaginary line (L1) and a focus (Fs) for the first reflection surface (16) are defined as second imaginary lines (L2), and a line formed by intersection points between a second reflection surface (17) and all the second imaginary lines (L2) is defined as a third imaginary line (L3), the adhesive (20) is, on an inner circumferential side relative to the ultrasonic wave generation source (11), provided in a region on an outer circumferential side relative to the third imaginary line (L3).

## Description

### TECHNICAL FIELD

The present invention relates to an ultrasonic wave generation device and an ultrasonic wave generation system.

### BACKGROUND ART

Conventionally, ultrasonic wave generation devices used for various purposes such as diagnosis and treatment have been known. For example, the following Patent Document 1 describes an ultrasonic wave generation device including: an ultrasonic wave generation source which generates an ultrasonic wave; an ultrasonic wave converging part which converges the ultrasonic wave generated by the ultrasonic wave generation source; and a waveguide which allows transmission of the ultrasonic wave converged by the ultrasonic wave converging part. The ultrasonic wave generated by the ultrasonic wave generation source is transmitted to the front end of the waveguide.

### PRIOR ART DOCUMENT

### PATENT DOCUMENT

Patent Document 1: WO2006/028249

### SUMMARY OF THE INVENTION

### PROBLEM TO BE SOLVED BY THE INVENTION

In general, an ultrasonic wave generation source of an ultrasonic wave generation device such as one described above is joined to an ultrasonic wave converging part by using an adhesive or the like. In this case, it is desirable that the adhesive is evenly spread on adhesion surfaces of the ultrasonic wave generation source and the ultrasonic wave converging part. However, if the adhesive sticks out from the adhesion surfaces, the intensity of an ultrasonic wave might decrease owing to the adhesive sticking out.

The present invention has been completed in view of the above circumstance, and an object of the present invention is to provide an ultrasonic wave generation device and an ultrasonic wave generation system that can inhibit the intensity of an ultrasonic wave from decreasing owing to an adhesive.

### MEANS FOR SOLVING THE PROBLEM

An ultrasonic wave generation device of present invention 1 includes: an ultrasonic wave generation source configured to generate an ultrasonic wave; an ultrasonic wave converging part configured to converge the ultrasonic wave generated from the ultrasonic wave generation source; and a waveguide configured to allow transmission therethrough of the ultrasonic wave converged by the ultrasonic wave converging part. The ultrasonic wave converging part has a first reflection surface located to be opposed to the ultrasonic wave generation source and a second reflection surface located to be opposed to the first reflection surface. The first reflection surface is a curved surface protruding to a side opposite to the ultrasonic wave generation source and reflects the ultrasonic wave that has been generated from the ultrasonic wave generation source, toward the second reflection surface. The second reflection surface is a curved surface protruding to a side opposite to the first reflection surface and reflects the ultrasonic wave that has been reflected by the first reflection surface, toward the waveguide so as to introduce the ultrasonic wave into the waveguide. The ultrasonic wave generation source encloses a circumference, of the second reflection surface, in a radial direction orthogonal to a direction in which the ultrasonic wave generation source and the first reflection surface are opposed to each other. The ultrasonic wave converging part is adhered to the ultrasonic wave generation source by using an adhesive. When a line obtained by projecting an outer circumferential edge of the ultrasonic wave generation source onto the first reflection surface along an advancing direction of the ultrasonic wave generated from the ultrasonic wave generation source is defined as a first imaginary line, and straight lines passing through points on the first imaginary line and a focus for the first reflection surface are defined as second imaginary lines, and a line formed by intersection points between the second reflection surface and all the second imaginary lines is defined as a third imaginary line, the adhesive is, on an inner circumferential side relative to the ultrasonic wave generation source, provided in a region on an outer circumferential side relative to the third imaginary line.

An ultrasonic wave generation device of present invention 2 includes: an ultrasonic wave generation source configured to generate an ultrasonic wave; an ultrasonic wave converging part configured to converge the ultrasonic wave generated from the ultrasonic wave generation source; and a waveguide configured to allow transmission therethrough of the ultrasonic wave converged by the ultrasonic wave converging part. The ultrasonic wave converging part has a first reflection surface located to be opposed to the ultrasonic wave generation source and a second reflection surface located to be opposed to the first reflection surface. The first reflection surface is a curved surface protruding to a side opposite to the ultrasonic wave generation source and reflects the ultrasonic wave that has been generated from the ultrasonic wave generation source, toward the second reflection surface. The second reflection surface is a curved surface protruding to a side opposite to the first reflection surface and reflects the ultrasonic wave that has been reflected by the first reflection surface, toward the waveguide so as to introduce the ultrasonic wave into the waveguide. The ultrasonic wave generation source encloses a circumference, of the second reflection surface, in a radial direction orthogonal to a direction in which the ultrasonic wave generation source and the first reflection surface are opposed to each other. The ultrasonic wave converging part is adhered to the ultrasonic wave generation source by using an adhesive. When a line obtained by projecting an outer circumferential edge of the ultrasonic wave generation source onto the first reflection surface along an advancing direction of the ultrasonic wave generated from the ultrasonic wave generation source is defined as a first imaginary line, the adhesive adhering the ultrasonic wave converging part and the ultrasonic wave generation source to each other is provided also on an outer circumferential side relative to the ultrasonic wave generation source and is not provided on an inner circumferential side of the first reflection surface relative to the first imaginary line.

### ADVANTAGEOUS EFFECTS OF THE INVENTION

According to present invention 1, the adhesive is not provided in a region, of the second reflection surface, on the inner circumferential side relative to the third imaginary line (a region, of the second reflection surface, in which a particularly intense ultrasonic wave is applied). Therefore, the intensity of an ultrasonic wave can be inhibited from decreasing owing to the adhesive.

According to present invention 2, the adhesive is not provided in a region, of the first reflection surface, on the inner circumferential side relative to a first imaginary plane (a region, of the first reflection surface, in which a particularly intense ultrasonic wave is applied). Therefore, the intensity of an ultrasonic wave can be inhibited from decreasing owing to the adhesive.

### BRIEF DESCRIPTION OF THE DRAWINGS

[Fig. 1] Diagram for explaining a basic configuration of an ultrasonic wave generation device and showing an image in which an ultrasonic wave generated from an ultrasonic wave generation source advances straight toward a first reflection surface.
[Fig. 2] Diagram for explaining the basic configuration of the ultrasonic wave generation device and showing an image in which the ultrasonic wave reflected by the first reflection surface heads for and is converged to a focus.
[Fig. 3] Schematic cross-sectional view showing an ultrasonic wave generation device in embodiment 1.
[Fig. 4] Bottom view of the ultrasonic wave generation device as seen from below.
[Fig. 5] Schematic cross-sectional view showing an ultrasonic wave generation device in embodiment 2.
[Fig. 6] Schematic cross-sectional view showing an ultrasonic wave generation device in embodiment 3.
[Fig. 7] Schematic cross-sectional view showing an ultrasonic wave generation device in embodiment 4.
[Fig. 8] Diagram showing a configuration of an ultrasonic wave generation system in embodiment 5.
[Fig. 9] Schematic cross-sectional view showing an ultrasonic wave generation device in another embodiment (3).

### MODES FOR CARRYING OUT THE INVENTION

Preferable modes of the present invention will be described below.

The ultrasonic wave generation device of present invention 1 may be configured such that: an accumulation space for the adhesive is provided between an inner circumferential edge of the ultrasonic wave generation source and an outer circumferential edge of the second reflection surface; and the accumulation space extends along an adhesion surface and has a size that allows the adhesive to be accumulated in the accumulation space. With such a configuration, the adhesive sticking out to the inner circumferential side of the ultrasonic wave generation source is accumulated in the accumulation space, whereby the adhesive can be made less likely to be adhered to the second reflection surface than in a case where the accumulation space is not provided.

Alternatively, the ultrasonic wave generation device of present invention 1 may be configured such that a step surface extending in a direction intersecting with an adhesion surface is formed between an inner circumferential edge of the ultrasonic wave generation source and an outer circumferential edge of the second reflection surface. With such a configuration, the adhesive sticking out to the inner circumferential side of the ultrasonic wave generation source is adhered to the step surface, whereby the adhesive can be made less likely to be adhered to the second reflection surface than in a case where the step surface is not provided.

Alternatively, the ultrasonic wave generation device of present invention 1 may be configured such that a wall protruding in a direction intersecting with an adhesion surface is provided between an inner circumferential edge of the ultrasonic wave generation source and an outer circumferential edge of the second reflection surface. With such a configuration, the adhesive sticking out from the inner circumferential edge of the ultrasonic wave generation source stays on the ultrasonic wave generation source side owing to the wall, whereby the adhesive can be made less likely to be adhered to the second reflection surface than in a case where the wall is not provided.

Alternatively, the ultrasonic wave generation device of present invention 1 may be configured such that a groove recessed in a direction intersecting with an adhesion surface is provided between an inner circumferential edge of the ultrasonic wave generation source and an outer circumferential edge of the second reflection surface. With such a configuration, the adhesive sticking out from the inner circumferential edge of the ultrasonic wave generation source stays on the ultrasonic wave generation source side owing to the groove, whereby the adhesive can be made less likely to be adhered to the second reflection surface than in a case where the groove is not provided.

In addition, the ultrasonic wave generation device of present invention 1 may be configured such that the groove is formed on the ultrasonic wave generation source side relative to an imaginary plane formed between the first imaginary line and the focus by all the second imaginary lines. With such a configuration, the groove does not overlap with a propagation region for an ultrasonic wave converging from the first reflection surface to the focus. Therefore, the groove does not hinder propagation of the ultrasonic wave converging from the first reflection surface to the focus.

The ultrasonic wave generation device of present invention 2 may be configured such that a wall protruding in a direction intersecting with an adhesion surface is provided on the outer circumferential side relative to the outer circumferential edge of the ultrasonic wave generation source. With such a configuration, the adhesive sticking out from the outer circumferential edge of the ultrasonic wave generation source stays on the ultrasonic wave generation source side owing to the wall, whereby the adhesive can be made less likely to be adhered to the first reflection surface than in a case where the wall is not provided.

The ultrasonic wave generation device of present invention 2 may be configured such that a groove recessed in a direction intersecting with an adhesion surface is provided on the outer circumferential side relative to the outer circumferential edge of the ultrasonic wave generation source. With such a configuration, the adhesive sticking out from the outer circumferential edge of the ultrasonic wave generation source stays on the ultrasonic wave generation source side owing to the groove, whereby the adhesive can be made less likely to be adhered to the first reflection surface than in a case where the groove is not provided.

<Embodiment 1>

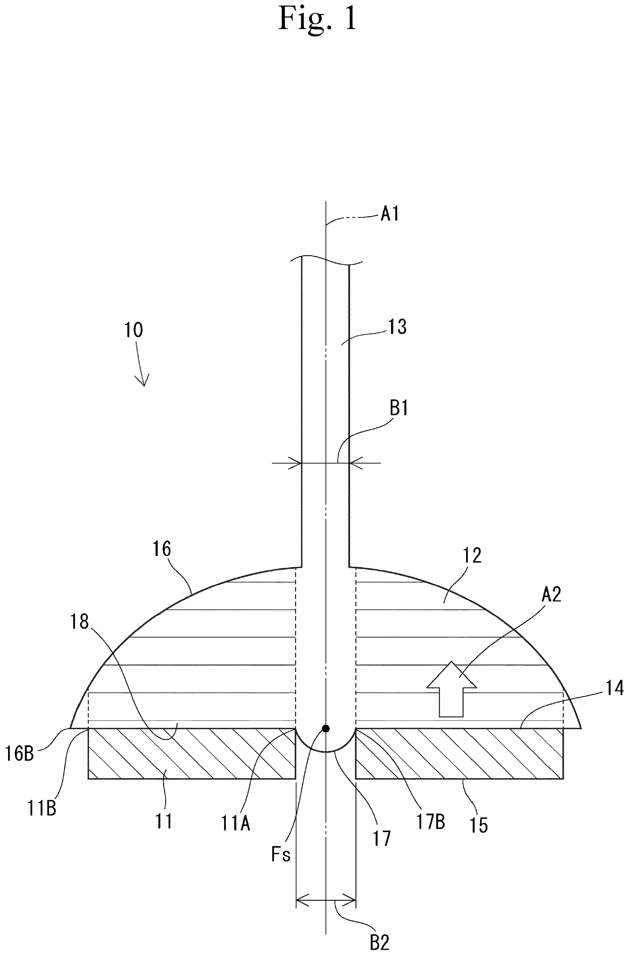

Hereinafter, an embodiment of the present invention will be described in detail with reference to Fig. 1 to Fig. 4. An ultrasonic wave generation device 10 in the present embodiment is used for an ultrasonograph, an ultrasonic treatment device, a cavitation generator, or the like.

Firstly, a basic configuration of the ultrasonic wave generation device 10 will be described. As shown in Fig. 1, the ultrasonic wave generation device 10 includes an ultrasonic wave generation source 11, an ultrasonic wave converging part 12, and a waveguide 13. The ultrasonic wave generation source 11 generates an ultrasonic wave. The ultrasonic wave converging part 12 converges the ultrasonic wave generated from the ultrasonic wave generation source 11. The waveguide 13 allows transmission therethrough of the ultrasonic wave converged by the ultrasonic wave converging part 12. The ultrasonic wave transmitted through the waveguide 13 is applied to an object. The object is not particularly limited and may be, for example, the inside of a living body. The ultrasonic wave applied to the object is reflected by the object and returned to the inside of the waveguide 13 as an ultrasonic wave carrying image information about the object. The ultrasonic wave carrying the image information about the object is returned to the ultrasonic wave generation source 11 through the waveguide 13 and the ultrasonic wave converging part 12. An electric signal based on the ultrasonic wave carrying the image information is received by a signal transmission/reception circuit, and the image information included in the received signal is displayed on a signal display device. As a technology for displaying an image on the basis of such an ultrasonic wave including image information, a known technology employed for an ultrasonograph or the like can be employed.

The ultrasonic wave generation source 11 is, for example, a piezoelectric element. The ultrasonic wave generation source 11 is formed in the shape of a plate having a predetermined thickness dimension. The ultrasonic wave generation source 11 has a first main surface 14 and a second main surface 15 on a side opposite to the first main surface 14. Electrodes (not shown) are disposed on the first main surface 14 and the second main surface 15.

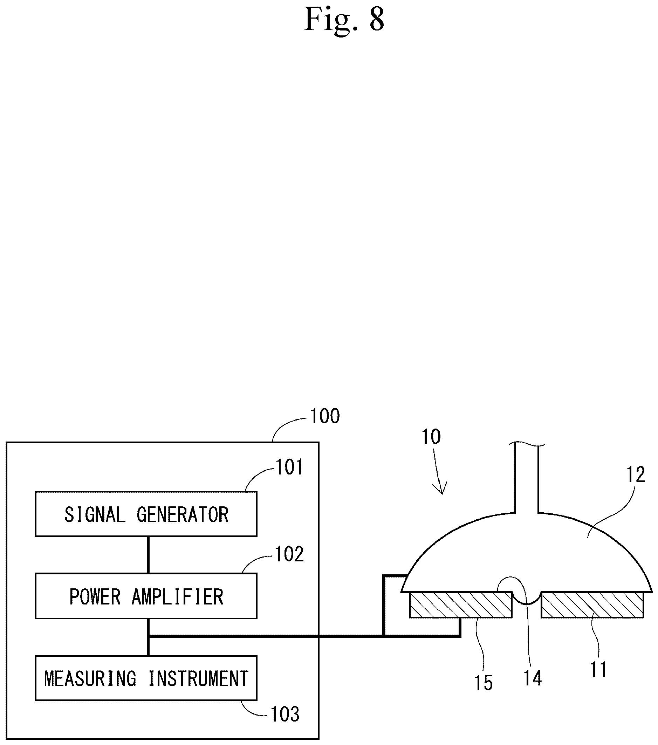

The ultrasonic wave generation source 11 generates an ultrasonic wave when receiving an electric signal from a power supply device 50 (see Fig. 8). The ultrasonic wave generated from the ultrasonic wave generation source 11 is a plane wave that advances straight in a direction indicated by an arrow A2 as shown in Fig. 1. The arrow A2 indicates the advancing direction of the ultrasonic wave generated from the ultrasonic wave generation source 11. The arrow A2 is parallel to an axis A1. The ultrasonic wave generation source 11 generates an ultrasonic wave having, for example, a frequency equal to or higher than 30 kHz. Hereinafter, regarding each constituent member, the direction indicated by the arrow A2 in Fig. 1 is defined as an upward direction, and the direction opposite thereto is defined as a downward direction.

The ultrasonic wave converging part 12 is formed of a solid metal (for example, duralumin). The ultrasonic wave converging part 12 has a first reflection surface 16 and a second reflection surface 17. The first reflection surface 16 is located to be opposed to the ultrasonic wave generation source 11. A direction in which the first reflection surface 16 and the ultrasonic wave generation source 11 are opposed to each other is parallel to the direction in which the axis A1 extends. The first reflection surface 16 is a paraboloid protruding to the upper side (the side opposite to the ultrasonic wave generation source 11) as seen from outside of the ultrasonic wave converging part 12. The first reflection surface 16 has a recessed shape as seen from inside of the ultrasonic wave converging part 12. A center portion of the first reflection surface 16 is located above an outer circumferential edge 16B of the first reflection surface 16. The first reflection surface 16 is a paraboloid of revolution about the axis A1.

The second reflection surface 17 is located to be opposed to the first reflection surface 16. The second reflection surface 17 is a paraboloid protruding to the lower side (the side opposite to the first reflection surface 16) as seen from outside of the ultrasonic wave converging part 12. The second reflection surface 17 has a recessed shape as seen from inside of the ultrasonic wave converging part 12. A center portion of the second reflection surface 17 is located below an outer circumferential edge 17B of the second reflection surface 17. The second reflection surface 17 is a paraboloid of revolution about the axis A1.

The waveguide 13 has the shape of a solid pillar. The waveguide 13 extends upward along the axis A1 from the center portion of the first reflection surface 16. A cross section, of the waveguide 13, orthogonal to the axis A1 has a circular shape about the axis A1. A diameter dimension B1, in a direction orthogonal to the axis A1, of the waveguide 13 is smaller than a diameter dimension B2, in the direction orthogonal to the axis A1, of the second reflection surface 17.

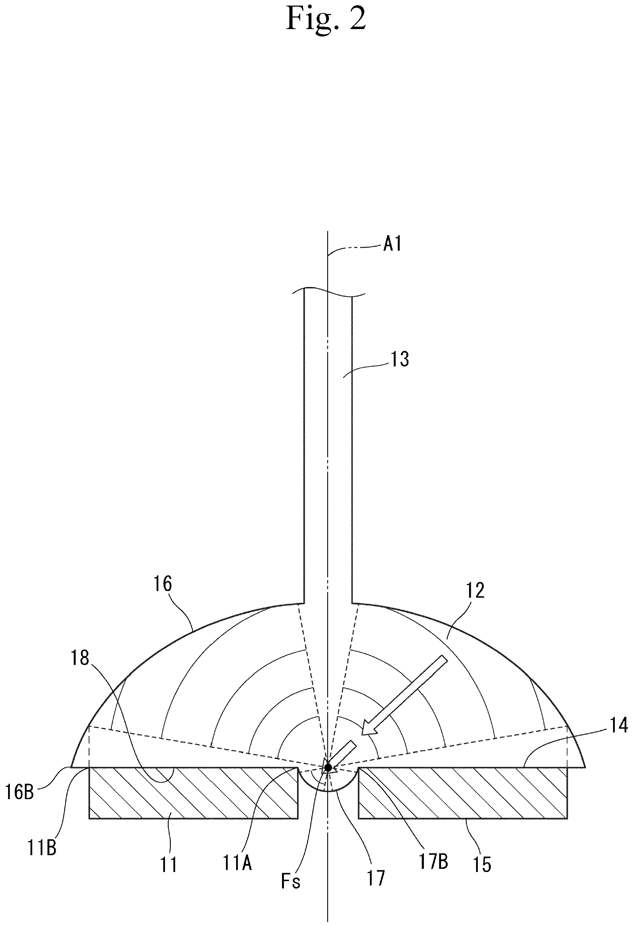

The ultrasonic wave generated from the ultrasonic wave generation source 11 is reflected by the first reflection surface 16 and converged to a focus Fs for the first reflection surface 16, as shown in Fig. 2. The ultrasonic wave having passed through the focus Fs is reflected by the second reflection surface 17 and converged to a focus Fs for the second reflection surface 17. The focus Fs for the second reflection surface 17 is identical to the focus Fs for the first reflection surface 16. The intensity of an ultrasonic wave per unit area of the second reflection surface 17 is higher than the intensity of an ultrasonic wave per unit area of the first reflection surface 16. The ultrasonic wave reflected by the second reflection surface 17 and having passed through the focus Fs is introduced into the waveguide 13 as a plane wave. The focus Fs is located on the axis A1.

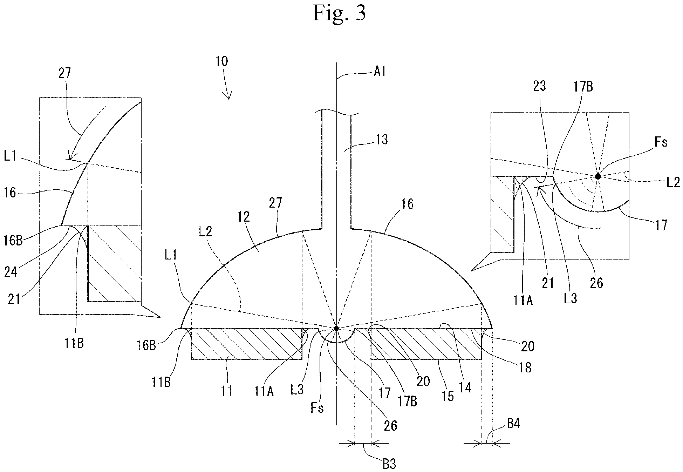

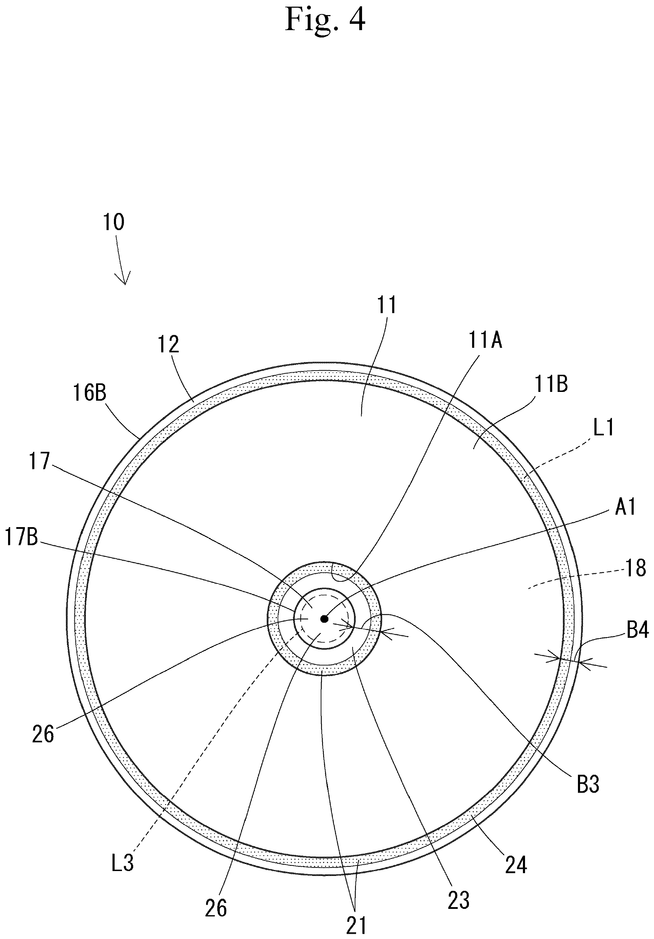

Next, the details of the ultrasonic wave generation device 10 will be described. The ultrasonic wave generation source 11 encloses a circumference of the second reflection surface 17 as shown in Fig. 3 and Fig. 4. The ultrasonic wave generation source 11 has an annular shape about the axis A1. An inner circumferential edge 11A of the ultrasonic wave generation source 11 is apart from the outer circumferential edge 17B of the second reflection surface 17 outward in a radial direction orthogonal to the axis A1. An outer circumferential edge 11B of the ultrasonic wave generation source 11 is apart from the outer circumferential edge 16B of the first reflection surface 16 inward in the radial direction orthogonal to the axis A1. The inner circumferential edge 11A and the outer circumferential edge 11B of the ultrasonic wave generation source 11 are the outer circumferential edge and the inner circumferential edge of the first main surface 14, respectively. The outer circumferential edge 16B of the first reflection surface 16 and the outer circumferential edge 17B of the second reflection surface 17 have circular shapes that are concentric about the axis A1. The area of the second reflection surface 17 is smaller than the area of the first reflection surface 16.

The ultrasonic wave converging part 12 has an adhesion surface 18 to be adhered to the ultrasonic wave generation source 11. The adhesion surface 18 spreads, outward in the radial direction orthogonal to the axis A1, from the outer circumferential edge 17B of the second reflection surface 17 to the outer circumferential edge 16B of the first reflection surface 16. The adhesion surface 18 is a flat surface orthogonal to the axis A1. The adhesion surface 18 has an annular shape so as to enclose the circumference of the second reflection surface 17, about the axis A1.

The ultrasonic wave converging part 12 is adhered to the ultrasonic wave generation source 11 by using an adhesive 20. The adhesive 20 has a characteristic of allowing propagation of an ultrasonic wave. The adhesive 20 includes: an adhesion layer (not shown) disposed between the first main surface 14 of the ultrasonic wave generation source 11 and the adhesion surface 18 of the ultrasonic wave converging part 12; and an excess portion 21 sticking out from the first main surface 14. The adhesion layer is thin and spreads over the entirety of the first main surface 14. Thus, the ultrasonic wave converging part 12 and the ultrasonic wave generation source 11 can be firmly adhered to each other. In the adhesion layer, air bubbles that are formed are desirably reduced as much as possible, but air bubbles may be contained to some extent. The excess portion 21 prevents stress from being concentrated on an outer edge portion of the adhesion layer. Consequently, the ultrasonic wave generation source 11 can be inhibited from peeling from the outer edge portion of the adhesion layer.

The ultrasonic wave converging part 12 has an inner accumulation space 23 and an outer accumulation space 24 as accumulation spaces for the adhesive 20. The inner accumulation space 23 and the outer accumulation space 24 extend along the adhesion surface 18. In other words, the inner accumulation space 23 and the outer accumulation space 24 are portions of the adhesion surface 18.

The inner accumulation space 23 is provided on the inner circumferential side relative to the ultrasonic wave generation source 11. The inner accumulation space 23 is a portion, of the adhesion surface 18, that is located between the inner circumferential edge 11A of the ultrasonic wave generation source 11 and the outer circumferential edge 17B of the second reflection surface 17. The inner accumulation space 23 has an annular shape about the axis A1 as shown in Fig. 4. The inner accumulation space 23 encloses the outer circumferential side of the second reflection surface 17. A width dimension (a diameter dimension in the direction orthogonal to the axis A1) B3 of the inner accumulation space 23 is fixed over the entire circumference thereof. The inner accumulation space 23 has a size that allows the corresponding excess portion 21 of the adhesive 20 to be accumulated therein.

The outer accumulation space 24 is provided on the outer circumferential side relative to the ultrasonic wave generation source 11. The outer accumulation space 24 is a portion, of the adhesion surface 18, that is located between the outer circumferential edge 11B of the ultrasonic wave generation source 11 and the outer circumferential edge 16B of the first reflection surface 16. The outer accumulation space 24 has an annular shape about the axis A1 as shown in Fig. 4. The outer accumulation space 24 encloses the outer circumferential side of the ultrasonic wave generation source 11. A width dimension (a diameter dimension in the direction orthogonal to the axis A1) B4 of the outer accumulation space 24 is fixed over the entire circumference thereof. The outer accumulation space 24 has a size that allows the corresponding excess portion 21 of the adhesive 20 to be accumulated therein.

In the inner accumulation space 23, the corresponding excess portion 21 of the adhesive 20 stays. Thus, the excess portion 21 of the adhesive 20 is not provided in a specific region 26 of the second reflection surface 17. The specific region 26 of the second reflection surface 17 is a region, of the second reflection surface 17, in which a particularly intense ultrasonic wave is applied.

In the outer accumulation space 24, the corresponding excess portion 21 of the adhesive 20 stays. Thus, the excess portion 21 of the adhesive 20 is not provided in a specific region 27 of the first reflection surface 16. The specific region 27 of the first reflection surface 16 is a region, of the first reflection surface 16, in which a particularly intense ultrasonic wave is applied.

The specific region 26 of the second reflection surface 17 and the specific region 27 of the first reflection surface 16 will be described by defining a first imaginary line L1, second imaginary lines L2, and a third imaginary line L3. The first imaginary line L1 is an imaginary line on the first reflection surface 16.

The first imaginary line L1 is a line obtained by projecting the outer circumferential edge 11B of the ultrasonic wave generation source 11 onto the first reflection surface 16 along the advancing direction of the ultrasonic wave generated from the ultrasonic wave generation source 11. The first imaginary line L1 overlaps with the outer circumferential edge 11B of the ultrasonic wave generation source 11, as seen from below the ultrasonic wave generation device 10. The first imaginary line L1 has a circular shape about the axis A1 on the first reflection surface 16. The specific region 27 of the first reflection surface 16 is a region on the inner circumferential side of the first reflection surface 16 relative to the first imaginary line L1.

The second imaginary lines L2 are straight lines passing through points on the first imaginary line L1 and the focus Fs for the first reflection surface 16. Each of the second imaginary lines L2 extends obliquely downward from the first imaginary line L1 toward the focus Fs as shown in Fig. 3.

The third imaginary line L3 is an imaginary line on the second reflection surface 17. The third imaginary line L3 is formed by intersection points between the second reflection surface 17 and all the second imaginary lines L2. The third imaginary line L3 has a circular shape about the axis A1 on the second reflection surface 17. The third imaginary line L3 is located on the inner circumferential side relative to the outer circumferential edge 17B of the second reflection surface 17. The specific region 26 of the second reflection surface 17 is a region, of the second reflection surface 17, that is located inward of the third imaginary line L3.

Next, actions and effects of the embodiment configured as described above will be described. The ultrasonic wave generation device 10 in embodiment 1 includes the ultrasonic wave generation source 11, the ultrasonic wave converging part 12, and the waveguide 13. The ultrasonic wave generation source 11 generates an ultrasonic wave. The ultrasonic wave converging part 12 converges the ultrasonic wave generated from the ultrasonic wave generation source 11. The waveguide 13 allows transmission therethrough of the ultrasonic wave converged by the ultrasonic wave converging part 12. The ultrasonic wave converging part 12 has the first reflection surface 16 having a parabolic shape and the second reflection surface 17 having a parabolic shape. The first reflection surface 16 is located to be opposed to the ultrasonic wave generation source 11. The second reflection surface 17 is located to be opposed to the first reflection surface 16. The first reflection surface 16 reflects the ultrasonic wave that has been generated from the ultrasonic wave generation source 11, toward the second reflection surface 17. The second reflection surface 17 reflects the ultrasonic wave that has been reflected by the first reflection surface 16, toward the waveguide 13 so as to introduce the ultrasonic wave into the waveguide 13. The ultrasonic wave generation source 11 encloses a circumference, of the second reflection surface 17, in the radial direction orthogonal to the direction in which the ultrasonic wave generation source 11 and the first reflection surface 16 are opposed to each other. The ultrasonic wave converging part 12 is adhered to the ultrasonic wave generation source 11 by using the adhesive 20. When a line obtained by projecting the outer circumferential edge 11B of the ultrasonic wave generation source 11 onto the first reflection surface 16 along the advancing direction of the ultrasonic wave generated from the ultrasonic wave generation source 11 is defined as the first imaginary line L1, and straight lines passing through points on the first imaginary line L1 and the focus Fs for the first reflection surface 16 are defined as the second imaginary lines L2, and a line formed by the intersection points between the second reflection surface 17 and all the second imaginary lines L2 is defined as the third imaginary line L3, the adhesive 20 is, on the inner circumferential side relative to the ultrasonic wave generation source 11, provided in a region on the outer circumferential side relative to the third imaginary line L3.

With this configuration, the adhesive 20 is not provided in a region, of the second reflection surface 17, on the inner circumferential side relative to the third imaginary line L3 (a region, of the second reflection surface 17, in which a particularly intense ultrasonic wave is applied). Therefore, the intensity of an ultrasonic wave can be inhibited from decreasing owing to the adhesive 20.

In addition, the adhesive 20 adhering the ultrasonic wave converging part 12 and the ultrasonic wave generation source 11 to each other is provided also on the outer circumferential side relative to the ultrasonic wave generation source 11. The adhesive 20 is not provided on the inner circumferential side of the first reflection surface 16 relative to the first imaginary line L1. With this configuration, the adhesive 20 is not provided in a region, of the first reflection surface 16, in which a particularly intense ultrasonic wave is applied, whereby the intensity of an ultrasonic wave can be inhibited from decreasing owing to the adhesive 20.

In addition, the inner accumulation space 23 for the adhesive 20 is provided between the inner circumferential edge 11A of the ultrasonic wave generation source 11 and the outer circumferential edge 17B of the second reflection surface 17. The inner accumulation space 23 extends along the adhesion surface 18 and has a size that allows the adhesive 20 to be accumulated in the inner accumulation space 23. With this configuration, the adhesive 20 sticking out to the inner circumferential side of the ultrasonic wave generation source 11 is accumulated in the inner accumulation space 23, whereby the adhesive 20 can be made less likely to be adhered to the second reflection surface 17.

In addition, the outer accumulation space 24 for the adhesive 20 is provided between the outer circumferential edge 11B of the ultrasonic wave generation source 11 and the outer circumferential edge 16B of the first reflection surface 16. The outer accumulation space 24 extends along the adhesion surface 18 and has a size that allows the adhesive 20 to be accumulated in the outer accumulation space 24. With this configuration, the adhesive 20 sticking out from the outer circumferential side of the ultrasonic wave generation source 11 is accumulated in the outer accumulation space 24, whereby the adhesive 20 can be made less likely to be adhered to the first reflection surface 16.

### <Embodiment 2>

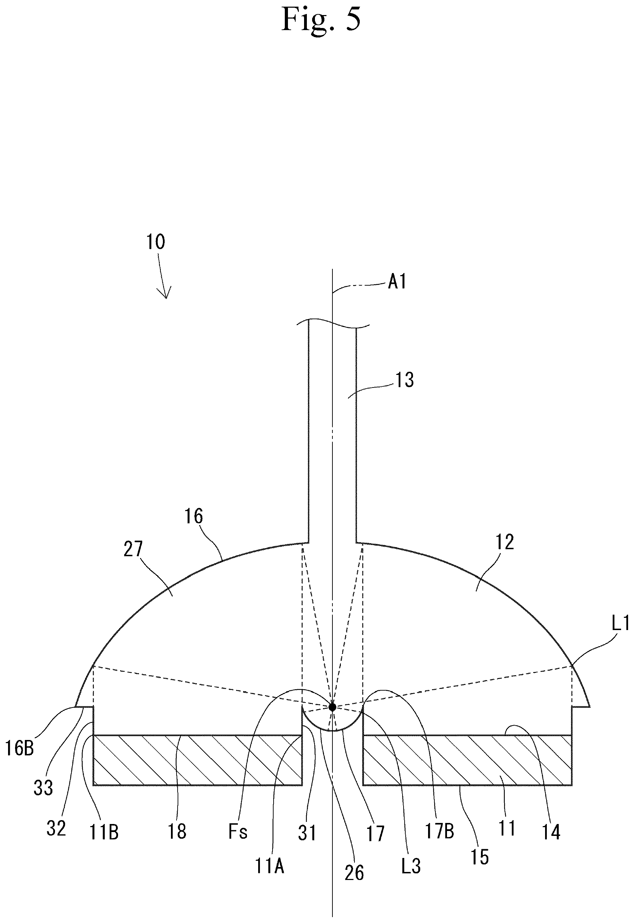

Next, an ultrasonic wave generation device 10 according to embodiment 2 of the present invention will be described with reference to Fig. 5. The ultrasonic wave generation device 10 in the present embodiment differs from that in embodiment 1 in that an inner step surface 31 and an outer step surface 32 are formed on the inner circumferential side and the outer circumferential side relative to the ultrasonic wave generation source 11. The same components as those in embodiment 1 are denoted by the same reference characters, and repetitive descriptions thereof are omitted.

Similar to embodiment 1, the ultrasonic wave generation device according to the present embodiment includes: the ultrasonic wave generation source 11 which generates an ultrasonic wave; the ultrasonic wave converging part 12 which converges the ultrasonic wave generated from the ultrasonic wave generation source 11; and the waveguide 13 which allows transmission therethrough of the ultrasonic wave converged by the ultrasonic wave converging part 12. In addition, similar to embodiment 1, the excess portion (not shown) of the adhesive 20, on the inner circumferential side relative to the ultrasonic wave generation source 11, is provided in the region on the outer circumferential side relative to the third imaginary line L3, and the excess portion (not shown) of the adhesive 20, on the outer circumferential side relative to the ultrasonic wave generation source 11, is not provided on the inner circumferential side of the first reflection surface 16 relative to the first imaginary line L1.

The inner step surface 31 is provided on the inner circumferential side relative to the ultrasonic wave generation source 11. The inner step surface 31 is provided at a center portion of the adhesion surface 18. The inner step surface 31 is provided between the inner circumferential edge 11A of the ultrasonic wave generation source 11 and the outer circumferential edge 17B of the second reflection surface 17. The inner step surface 31 extends upward from the adhesion surface 18 in a direction orthogonal to the adhesion surface 18. The inner step surface 31 has a cylindrical shape about the axis A1. The upper edge of the inner step surface 31 is the outer circumferential edge 17B of the second reflection surface 17. The lower edge of the inner step surface 31 is the inner circumferential edge 11A of the ultrasonic wave generation source 11.

The outer step surface 32 is provided on the outer circumferential side relative to the ultrasonic wave generation source 11. The outer step surface 32 is provided at an outer edge portion of the adhesion surface 18. The outer step surface 32 extends upward from the adhesion surface 18 in the direction orthogonal to the adhesion surface 18. The outer step surface 32 has a cylindrical shape about the axis A1. The upper edge of the outer step surface 32 is located inward of the outer circumferential edge 16B of the first reflection surface 16. A space 33 is provided between the upper edge of the outer step surface 32 and the outer circumferential edge 16B of the first reflection surface 16. The space 33 has a flat surface orthogonal to the axis A1. The lower edge of the outer step surface 32 is the outer circumferential edge 11B of the ultrasonic wave generation source 11.

As described above, in the present embodiment, the inner step surface 31 and the outer step surface 32 each extending in a direction intersecting with the adhesion surface 18 are formed on the inner circumferential side and the outer circumferential side, respectively, relative to the ultrasonic wave generation source 11. With this configuration, the adhesive 20 sticking out to the inner circumferential side of the ultrasonic wave generation source 11 is adhered to the inner step surface 31, whereby the adhesive 20 can be made less likely to be adhered to the second reflection surface 17. Meanwhile, the adhesive 20 sticking out to the outer circumferential side of the ultrasonic wave generation source 11 is adhered to the outer step surface 32, whereby the adhesive 20 can be made less likely to be adhered to the first reflection surface 16.

Consequently, similar to embodiment 1, the adhesive 20 is, on the inner circumferential side relative to the ultrasonic wave generation source 11, provided in the region on the outer circumferential side relative to the third imaginary line L3, and the adhesive 20 is, on the outer circumferential side relative to the ultrasonic wave generation source 11, not provided on the inner circumferential side of the first reflection surface 16 relative to the first imaginary line L1. Therefore, the intensity of an ultrasonic wave can be inhibited from decreasing owing to the adhesive 20.

### <Embodiment 3>

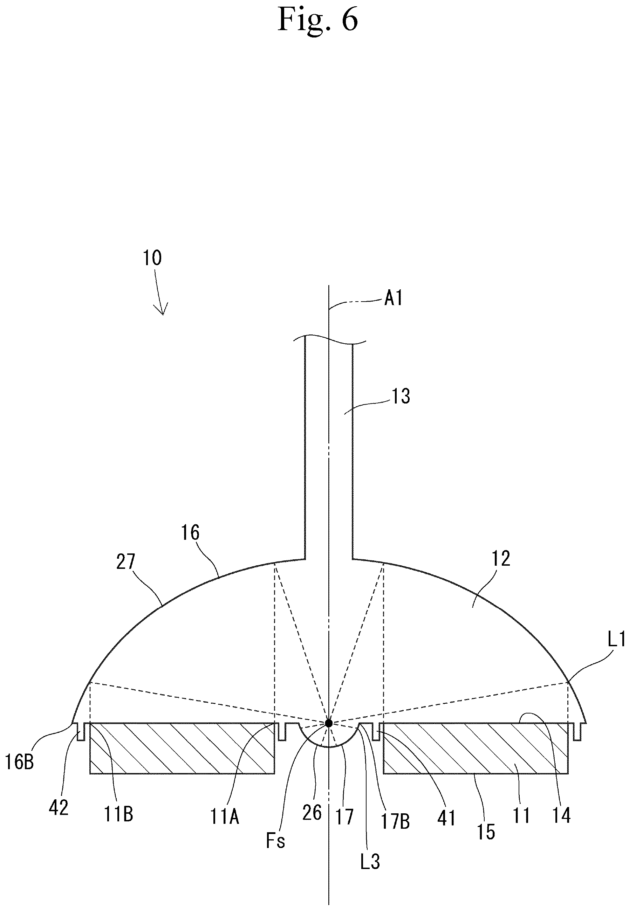

Next, an ultrasonic wave generation device 10 according to embodiment 3 of the present invention will be described with reference to Fig. 6. The ultrasonic wave generation device 10 in the present embodiment differs from that in embodiment 1 in that an inner wall 41 and an outer wall 42 are provided on the inner circumferential side and the outer circumferential side, respectively, relative to the ultrasonic wave generation source 11. The same components as those in embodiment 1 are denoted by the same reference characters, and repetitive descriptions thereof are omitted.

Similar to embodiment 1, the ultrasonic wave generation device according to the present embodiment includes: the ultrasonic wave generation source 11 which generates an ultrasonic wave; the ultrasonic wave converging part 12 which converges the ultrasonic wave generated from the ultrasonic wave generation source 11; and the waveguide 13 which allows transmission therethrough of the ultrasonic wave converged by the ultrasonic wave converging part 12. In addition, similar to embodiment 1, the excess portion (not shown) of the adhesive 20, on the inner circumferential side relative to the ultrasonic wave generation source 11, is provided in the region on the outer circumferential side relative to the third imaginary line L3, and the excess portion (not shown) of the adhesive 20, on the outer circumferential side relative to the ultrasonic wave generation source 11, is not provided on the inner circumferential side of the first reflection surface 16 relative to the first imaginary line L1.

The inner wall 41 is provided on the inner circumferential side relative to the ultrasonic wave generation source 11. The inner wall 41 is provided at the center portion of the adhesion surface 18. The inner wall 41 protrudes downward from the adhesion surface 18 in the direction orthogonal to the adhesion surface 18. The inner wall 41 is provided between the inner circumferential edge 11A of the ultrasonic wave generation source 11 and the outer circumferential edge 17B of the second reflection surface 17. The inner wall 41 has a cylindrical shape about the axis A1. The inner wall 41 is apart inward from the inner circumferential edge 11A of the ultrasonic wave generation source 11. The inner wall 41 is apart outward from the outer circumferential edge 17B of the second reflection surface 17.

The outer wall 42 is provided on the outer circumferential side relative to the ultrasonic wave generation source 11. The outer wall 42 is provided at the outer edge portion of the adhesion surface 18. The outer wall 42 protrudes downward from the adhesion surface 18 in the direction orthogonal to the adhesion surface 18. The outer wall 42 has a cylindrical shape about the axis A1. The outer wall 42 is apart outward from the outer circumferential edge 11B of the ultrasonic wave generation source 11. The outer wall 42 is apart inward from the outer circumferential edge 16B of the first reflection surface 16.

As described above, in the present embodiment, the inner wall 41 and the outer wall 42 each protruding in the direction intersecting with the adhesion surface 18 are formed on the inner circumferential side and the outer circumferential side, respectively, relative to the ultrasonic wave generation source 11. With this configuration, the adhesive 20 sticking out to the inner circumferential side of the ultrasonic wave generation source 11 stays on the ultrasonic wave generation source 11 side owing to the inner wall 41, whereby the adhesive 20 can be made less likely to be adhered to the second reflection surface 17. Meanwhile, the adhesive 20 sticking out to the outer circumferential side of the ultrasonic wave generation source 11 stays on the ultrasonic wave generation source 11 side owing to the outer wall 42, whereby the adhesive 20 can be made less likely to be adhered to the first reflection surface 16.

Consequently, similar to embodiment 1, the adhesive 20 is, on the inner circumferential side relative to the ultrasonic wave generation source 11, provided in the region on the outer circumferential side relative to the third imaginary line L3, and the adhesive 20 is, on the outer circumferential side relative to the ultrasonic wave generation source 11, not provided on the inner circumferential side of the first reflection surface 16 relative to the first imaginary line L1. Therefore, the intensity of an ultrasonic wave can be inhibited from decreasing owing to the adhesive 20.

### <Embodiment 4>

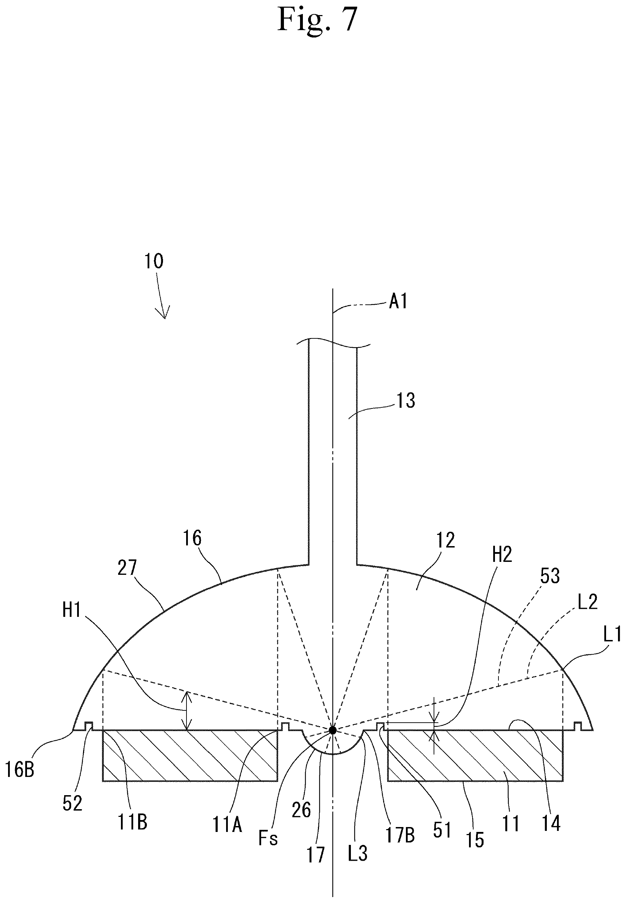

Next, an ultrasonic wave generation device 10 according to embodiment 4 of the present invention will be described with reference to Fig. 7. The ultrasonic wave generation device 10 in the present embodiment differs from that in embodiment 1 in that an inner groove 51 and an outer groove 52 are provided on the inner circumferential side and the outer circumferential side, respectively, relative to the ultrasonic wave generation source 11. The same components as those in embodiment 1 are denoted by the same reference characters, and repetitive descriptions thereof are omitted.

Similar to embodiment 1, the ultrasonic wave generation device 10 according to the present embodiment includes: the ultrasonic wave generation source 11 which generates an ultrasonic wave; the ultrasonic wave converging part 12 which converges the ultrasonic wave generated from the ultrasonic wave generation source 11; and the waveguide 13 which allows transmission therethrough of the ultrasonic wave converged by the ultrasonic wave converging part 12. In addition, similar to embodiment 1, the excess portion 21 (not shown) of the adhesive 20, on the inner circumferential side relative to the ultrasonic wave generation source 11, is provided in the region on the outer circumferential side relative to the third imaginary line L3, and the excess portion 21 (not shown) of the adhesive 20, on the outer circumferential side relative to the ultrasonic wave generation source 11, is not provided on the inner circumferential side of the first reflection surface 16 relative to the first imaginary line L1.

The inner groove 51 is provided on the inner circumferential side relative to the ultrasonic wave generation source 11. The inner groove 51 is provided at the center portion of the adhesion surface 18. The inner groove 51 is recessed upward from the adhesion surface 18 in the direction orthogonal to the adhesion surface 18. The inner groove 51 is provided between the inner circumferential edge 11A of the ultrasonic wave generation source 11 and the outer circumferential edge 17B of the second reflection surface 17. The inner groove 51 has an annular shape about the axis A1. The inner groove 51 is apart inward from the inner circumferential edge 11A of the ultrasonic wave generation source 11. The inner groove 51 is apart outward from the outer circumferential edge 17B of the second reflection surface 17.

The inner groove 51 is formed on the lower side (the ultrasonic wave generation source 11 side) relative to an imaginary plane 53 formed by all the second imaginary lines L2 between the first imaginary line L1 and the focus Fs. The imaginary plane 53 has, in a side view, a right circular conical shape about the axis A1. A dimension H1 in the up-down direction parallel to the axis A1 from the adhesion surface 18 to the imaginary plane 53 gradually increases from the center side toward the outer circumferential side of the adhesion surface 18. A dimension (depth dimension) H2, in the up-down direction parallel to the axis A1, of the inner groove 51 is smaller than the dimension H1, in the up-down direction, of the imaginary plane 53 at the position of the inner groove 51.

The outer groove 52 is provided on the outer circumferential side relative to the ultrasonic wave generation source 11. The outer groove 52 is provided at the outer edge portion of the adhesion surface 18. The outer groove 52 is recessed upward from the adhesion surface 18 in the direction orthogonal to the adhesion surface 18. The outer groove 52 has an annular shape about the axis A1. The outer groove 52 is apart outward from the outer circumferential edge 11B of the ultrasonic wave generation source 11. The outer groove 52 is apart inward from the outer circumferential edge 16B of the first reflection surface 16.

As described above, in the present embodiment, similar to embodiment 1, the inner groove 51 and the outer groove 52 each recessed in the direction intersecting with the adhesion surface 18 are formed on the inner circumferential side and the outer circumferential side, respectively, relative to the ultrasonic wave generation source 11. With this configuration, the adhesive 20 sticking out to the inner circumferential side of the ultrasonic wave generation source 11 stays on the ultrasonic wave generation source 11 side owing to the inner groove 51, whereby the adhesive 20 can be made less likely to be adhered to the second reflection surface 17. Meanwhile, the adhesive 20 sticking out to the outer circumferential side of the ultrasonic wave generation source 11 stays on the ultrasonic wave generation source 11 side owing to the outer groove 52, whereby the adhesive 20 can be made less likely to be adhered to the first reflection surface 16.

Consequently, similar to embodiment 1, the adhesive 20 is, on the inner circumferential side relative to the ultrasonic wave generation source 11, provided in the region on the outer circumferential side relative to the third imaginary line L3, and the adhesive 20 is, on the outer circumferential side relative to the ultrasonic wave generation source 11, not provided on the inner circumferential side of the first reflection surface 16 relative to the first imaginary line L1. Therefore, the intensity of an ultrasonic wave can be inhibited from decreasing owing to the adhesive 20.

The inner groove 51 is formed on the ultrasonic wave generation source 11 side relative to the imaginary plane 53 formed by all the second imaginary lines L2 between the first imaginary line L1 and the focus Fs. With this configuration, the inner groove 51 does not overlap with a propagation region for an ultrasonic wave converging from the first reflection surface 16 to the focus Fs. Therefore, the inner groove 51 does not hinder propagation of the ultrasonic wave converging from the first reflection surface 16 to the focus Fs.

### <Embodiment 5>

Next, an ultrasonic wave generation system according to embodiment 5 of the present invention will be described with reference to Fig. 8. The same components as those in the above embodiments are denoted by the same reference characters, and repetitive descriptions thereof are omitted. The ultrasonic wave generation system in the present embodiment includes the ultrasonic wave generation device 10 and a power supply device 100. The power supply device 100 outputs an electric signal to each of the electrodes in the ultrasonic wave generation device 10 to drive the ultrasonic wave generation device 10.

The power supply device 100 includes a signal generator 101, a power amplifier 102, and a measuring instrument 103. The signal generator 101 generates an arbitrarily-set vibration signal. As the signal generator 101, for example, a frequency response analyzer may be used. The power amplifier 102 amplifies the signal outputted from the signal generator 101 and applies the amplified signal to the ultrasonic wave generation device 10. As the power amplifier 102, for example, a known power amplifier may be used. The measuring instrument 103 monitors the signal amplified by the power amplifier 102. As the measuring instrument 103, for example, an oscilloscope may be used. The power supply device 100 supplies the amplified signal to the ultrasonic wave generation device 10 and monitors applied voltage.

In the present embodiment, the ultrasonic wave converging part 12 is formed of a metal and has electrical conductivity, and thus the power supply device 100 is connected to the ultrasonic wave converging part 12 and electrically connected to the ultrasonic wave generation source 11 via the ultrasonic wave converging part 12. Therefore, if the ultrasonic wave converging part 12 is an insulating material, it is desirable that the power supply device 100 and the ultrasonic wave generation source 11 are connected to each other directly or via another electrically-conductive member.

### <Other Embodiments>

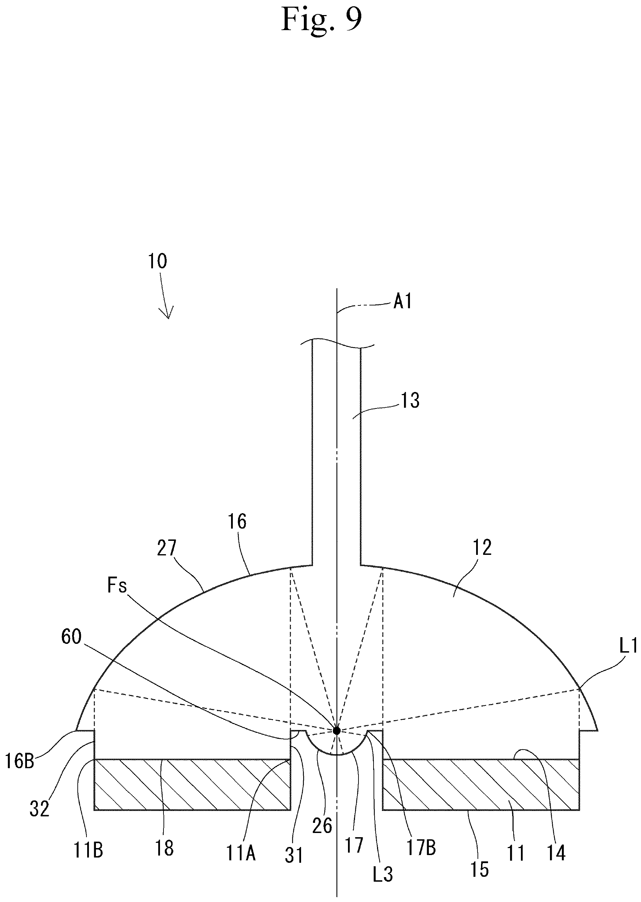

Embodiments of the present invention are not limited to those described in the above explanations and with reference to the drawings, and, for example, the following embodiments are also included in the technical scope of the present invention.
(1) In the above embodiment 1, the ultrasonic wave generation device 10 includes the inner accumulation space 23 and the outer accumulation space 24. Without limitation thereto, the ultrasonic wave generation device may include only one of the inner accumulation space and the outer accumulation space.
(2) In the above embodiment 2, the inner step surface 31 and the outer step surface 32 are orthogonal to the adhesion surface 18. Without limitation thereto, the step surfaces may be tilted with respect to the adhesion surface.
(3) In the above embodiment 2, the ultrasonic wave generation device 10 includes the inner step surface 31 and the outer step surface 32. The ultrasonic wave generation device 10 may be provided with, in addition to these step surfaces, a space 60 on the inner circumferential side relative to the inner step surface 31 as shown in Fig. 9. The space 60 may be formed as a flat surface orthogonal to the axis A1.
(4) In each of the above embodiments, the excess portion 21 of the adhesive 20 may be provided on the upper side relative to the outer circumferential edge 16B of the first reflection surface 16 and may be, if the amount thereof is small, provided on the inner circumferential side of the first reflection surface 16 relative to the first imaginary line L1.
(5) In each of the above embodiments, the first reflection surface 16 and the second reflection surface 17 are paraboloids. Without limitation thereto, both or one of the first reflection surface and the second reflection surface does not have to be a paraboloid in an exact sense and may have a shape with which the reflection surface can be regarded as an approximate paraboloid. In other words, both or one of the first reflection surface and the second reflection surface only has to be a surface that is curved such that the ultrasonic wave generated from the ultrasonic wave generation source reaches the waveguide via the first reflection surface and the second reflection surface. The first reflection surface and the second reflection surface may be composed of a large number of minute flat surfaces.
(6) In each of the above embodiments, the ultrasonic wave generation source 11 is a piezoelectric element formed of piezoelectric ceramic. Without limitation thereto, another piezoelectric material can be used for the ultrasonic wave generation source. The ultrasonic wave generation source may be, for example, a piezoelectric laminated body or the like formed of piezoelectric ceramic.
(7) In the above embodiments, the inner accumulation space 23 and the outer accumulation space 24, the inner step surface 31 and the outer step surface 32, the inner wall 41 and the outer wall 42, or the inner groove 51 and the outer groove 52 are respectively provided on the inner circumferential side and the outer circumferential side relative to the ultrasonic wave generation source 11. Without limitation thereto, components of different embodiments may be provided on the inner circumferential side and the outer circumferential side relative to the ultrasonic wave generation source. For example, a wall may be provided on the inner circumferential side and a groove may be provided on the outer circumferential side relative to the ultrasonic wave generation source.
(8) In each of the above embodiments, the waveguide 13 and the ultrasonic wave converging part 12 are each solid. However, the waveguide and the ultrasonic wave converging part are not limited to solid ones and may have, for example, through-holes or closed spaces formed therein.

### DESCRIPTION OF REFERENCE NUMERALS

Fs: focus
L1: first imaginary line
L2: second imaginary line
L3: third imaginary line
10: ultrasonic wave generation device
11: ultrasonic wave generation source
11A: inner circumferential edge of ultrasonic wave generation source
11B: outer circumferential edge of ultrasonic wave generation source
12: ultrasonic wave converging part
13: waveguide
16: first reflection surface
17: second reflection surface
17B: outer circumferential edge of second reflection surface
18: adhesion surface
20: adhesive
23: inner accumulation space (accumulation space)
31: inner step surface (step surface)
41: inner wall (wall)
42: outer wall (wall)
51: inner groove (groove)
52: outer groove (groove)
53: imaginary plane
100: power supply device

## Claims

1. An ultrasonic wave generation device comprising:
an ultrasonic wave generation source configured to generate an ultrasonic wave;
an ultrasonic wave converging part configured to converge the ultrasonic wave generated from the ultrasonic wave generation source; and
a waveguide configured to allow transmission therethrough of the ultrasonic wave converged by the ultrasonic wave converging part,
the ultrasonic wave converging part having a first reflection surface located to be opposed to the ultrasonic wave generation source and a second reflection surface located to be opposed to the first reflection surface,
the first reflection surface being a curved surface protruding to a side opposite to the ultrasonic wave generation source and configured to reflect the ultrasonic wave that has been generated from the ultrasonic wave generation source, toward the second reflection surface,
the second reflection surface being a curved surface protruding to a side opposite to the first reflection surface and configured to reflect the ultrasonic wave that has been reflected by the first reflection surface, toward the waveguide so as to introduce the ultrasonic wave into the waveguide, and
the ultrasonic wave generation source enclosing a circumference, of the second reflection surface, in a radial direction orthogonal to a direction in which the ultrasonic wave generation source and the first reflection surface are opposed to each other,
wherein
the ultrasonic wave converging part is adhered to the ultrasonic wave generation source by using an adhesive, and,
when a line obtained by projecting an outer circumferential edge of the ultrasonic wave generation source onto the first reflection surface along an advancing direction of the ultrasonic wave generated from the ultrasonic wave generation source is defined as a first imaginary line, and straight lines passing through points on the first imaginary line and a focus for the first reflection surface are defined as second imaginary lines, and a line formed by intersection points between the second reflection surface and all the second imaginary lines is defined as a third imaginary line,
the adhesive is, on an inner circumferential side relative to the ultrasonic wave generation source, provided in a region on an outer circumferential side relative to the third imaginary line.

2. An ultrasonic wave generation device comprising:
an ultrasonic wave generation source configured to generate an ultrasonic wave;
an ultrasonic wave converging part configured to converge the ultrasonic wave generated from the ultrasonic wave generation source; and
a waveguide configured to allow transmission therethrough of the ultrasonic wave converged by the ultrasonic wave converging part,
the ultrasonic wave converging part having a first reflection surface located to be opposed to the ultrasonic wave generation source and a second reflection surface located to be opposed to the first reflection surface,
the first reflection surface being a curved surface protruding to a side opposite to the ultrasonic wave generation source and configured to reflect the ultrasonic wave that has been generated from the ultrasonic wave generation source, toward the second reflection surface,
the second reflection surface being a curved surface protruding to a side opposite to the first reflection surface and configured to reflect the ultrasonic wave that has been reflected by the first reflection surface, toward the waveguide so as to introduce the ultrasonic wave into the waveguide, and
the ultrasonic wave generation source enclosing a circumference, of the second reflection surface, in a radial direction orthogonal to a direction in which the ultrasonic wave generation source and the first reflection surface are opposed to each other,
wherein
the ultrasonic wave converging part is adhered to the ultrasonic wave generation source by using an adhesive, and,
when a line obtained by projecting an outer circumferential edge of the ultrasonic wave generation source onto the first reflection surface along an advancing direction of the ultrasonic wave generated from the ultrasonic wave generation source is defined as a first imaginary line,
the adhesive adhering the ultrasonic wave converging part and the ultrasonic wave generation source to each other is provided also on an outer circumferential side relative to the ultrasonic wave generation source and is not provided on an inner circumferential side of the first reflection surface relative to the first imaginary line.

3. The ultrasonic wave generation device according to claim 1, wherein
an accumulation space for the adhesive is provided between an inner circumferential edge of the ultrasonic wave generation source and an outer circumferential edge of the second reflection surface, and
the accumulation space extends along an adhesion surface and has a size that allows the adhesive to be accumulated in the accumulation space.

4. The ultrasonic wave generation device according to claim 1, wherein
a step surface extending in a direction intersecting with an adhesion surface is formed between an inner circumferential edge of the ultrasonic wave generation source and an outer circumferential edge of the second reflection surface.

5. The ultrasonic wave generation device according to claim 1, wherein
a wall protruding in a direction intersecting with an adhesion surface is provided between an inner circumferential edge of the ultrasonic wave generation source and an outer circumferential edge of the second reflection surface.

6. The ultrasonic wave generation device according to claim 1, wherein
a groove recessed in a direction intersecting with an adhesion surface is provided between an inner circumferential edge of the ultrasonic wave generation source and an outer circumferential edge of the second reflection surface.

7. The ultrasonic wave generation device according to claim 6, wherein
the groove is formed on the ultrasonic wave generation source side relative to an imaginary plane formed by all the second imaginary lines between the first imaginary line and the focus.

8. The ultrasonic wave generation device according to claim 2, wherein
a wall protruding in a direction intersecting with an adhesion surface is provided on the outer circumferential side relative to the outer circumferential edge of the ultrasonic wave generation source.

9. The ultrasonic wave generation device according to claim 2, wherein
a groove recessed in a direction intersecting with an adhesion surface is provided on the outer circumferential side relative to the outer circumferential edge of the ultrasonic wave generation source.

10. An ultrasonic wave generation system comprising:
the ultrasonic wave generation device according to any one of claims 1 to 9; and
a power supply device configured to output an electric signal to the ultrasonic wave generation device.
